# EUROPEAN PATENT SPECIFICATION

(11) **EP 2 031 980 B1**
(45) Date of publication and mention of the grant of the patent: **02.06.2010**
(21) Application number: 07746477.4
(22) Date of filing: 11.05.2007
(51) Int. Cl.: A23L 1/212, A23L 1/30, A23G 3/00, A61K 36/258

(54) **METHOD OF MAKING A BLACK-GINSENG AND A CONDENSED BLACK-GINSENG AND A BLACK-GINSENG PRESERVED IN HONEY**
VERFAHREN ZUR HERSTELLUNG VON SCHWARZEM GINSENG, KONDENSIERTEM SCHWARZEM GINSENG UND IN HONIG KONSERVIERTEM SCHWARZEM GINSENG
PROCÉDÉ DE FABRICATION DE GINSENG NOIR, GINSENG NOIR CONCENTRÉ ET GINSENG NOIR ENROBÉ DE MIEL

(30) Priority: 12.05.2006 KR 20060043158; 24.06.2006 KR 20060057175; 01.07.2006 KR 20060061700
(43) Date of publication of application: 11.03.2009
(73) Proprietor: Lee, Jung Won, Jinan-gun, Jeollabuk-do 567-861 (KR)
(72) Inventor: Lee, Jung Won, Jinan-gun, Jeollabuk-do 567-861 (KR)
(74) Representative: Verscht, Thomas Kurt Albert
(86) International application number: PCT/KR2007/002328
(87) International publication number: WO 2007/133014

(56) References cited:
- WO-A1-2005/020715
- KR-A- 20020 097 099
- KR-A- 20030 005 089
- KR-A- 20040 023 185
- KR-A- 20040 090 285
- KR-B1- 100 543 862

## Description

### Technical Field

The present invention relates to the manufacture of black ginseng, a black ginseng extract and a honey-soaked black ginseng confectionary from raw fresh ginseng. Black ginseng is manufactured by conducting a cleaning process, a primary steaming process, a primary maturing process, a secondary steaming process, a secondary steaming process, a form fixing process, a tertiary ~ nonary steaming and tertiary ~ nonary maturing process, and a drying process, in that order. The black ginseng extract is manufactured by conducting a cleaning process, a primary steaming process, a primary maturing process, a secondary steaming process, a form fixing process, tertiary ~ nonary steaming and tertiary ~ nonary maturing processes, an extracting process, a filtering process, a concentrating process, a purifying process, and a concentrating process in that order. Thehoney-soaked black ginseng confectionary is manufactured by conducting a cleaning process, a surface dehydrating and skin peeling process, a neat arranging process, a honey soaking process, a primary steaming process, a primary maturing process, a honey soaking process, a secondary steaming process, a secondary maturing process, a honey soaking process, a form fixing process, tertiary - nonary steaming and tertiary - nonary maturing processes, a primary drying process, a pruning process, a secondary drying process and a packaging process.

### Background Art

Generally, red ginseng is prepared from fresh ginseng through washing, sorting and trimming, steaming, drying, selecting, inspecting and packaging, in that order. In almost all cases, the most important processes for the preparation of red ginseng, namely steaming and drying, are performed once.

For the preparation of sugar-soaked ginseng confectionary, a typical method comprises washing ginseng to remove foreign matter therefrom, peeling the ginseng, steaming the ginseng, soaking the ginseng with sugar, removing excess sugar, drying the ginseng in forced air, and packaging it.

As for black ginseng, the preparation thereof is largely dependent on steaming and drying processes. However, the large number of rounds of these processes causes ginseng ingredients to undergo pyrolysis or other chemical changes leading to carbonization. In addition, when it is exposed to low temperatures for a long period of time in the manufacturing processes thereof, the ginseng, having a high water content, is prone to deteriorate or lose effective ingredients as a result of germinal activity. Where black ginseng is prepared without the separation of the root body from the root hairs, a significant difference occurs in the rate and extent of drying between the root body and the root hairs. For example, if the root body of ginseng is dried to a water content of 14 % or lower, the root hairs become too dry. On the other hand, if a drying process is performed on the basis of the water content of root hairs, the root body contains too much water.

Conventional methods of preparing sugar-soaked ginseng confectionary are quite standardized, resulting in uniform tastes and flavors.

### Disclosure of Invention

### Technical Problem

Accordingly, the present invention has been made keeping in mind the above problems occurring in the prior art, and an object of the present invention is to provide methods for manufacturing black ginseng products, including black ginseng itself, black ginseng extracts and a honey-soaked black ginseng confectionary, which can be commercially valuable commoditiescompeting with conventional ginseng and red ginseng products, are healthful for the body, and have an excellent taste and flavor for persons of all ages all over the world.

It is another object of the present invention to provide methods for manufacturing black ginseng products, including black ginseng itself, black ginseng extracts and a honey-soaked black ginseng confectionary, by which the black ginseng can contain effective saponin ingredients without loss thereof attributable to carbonization, retain the original form of the starting fresh ginseng material, increase in value, and have water uniformly distributed throughout the root body and hairs.

It is a further object of the present invention to provide a method of manufacturing a black ginseng extract containing an effective saponin components in an amount of 80 mg/g - 300 mg/g.

It is a still further object of the present invention to provide a method of manufacturing a honey-soaked black ginseng confectionary which retains the effective ingredients of the starting material and has characteristic excellent taste and flavor.

### Technical Solution

In accordance with an aspect of the present invention, there is provided a method for manufacturing black ginseng from fresh ginseng, comprising: cleaning raw fresh ginseng with water carefully, soas not to injure the integrity and ingredients of the fresh ginseng; primarily steaming the cleaned ginseng at 88 - 96°C for 2.5 ~ 4 hours with the ginseng's own water in a steaming chamber with alcohol provided into the chamber; primarily maturing the ginseng at 35°Cor lower for 24 - 72 hours in a maturing chamber with no additional provision of water to the chamber in such a manner that the water content of the ginseng is not decreased below 20%; secondarily steaming the primarily matured ginseng at 65 ~ 75°C for 1.5 ~ 2.5 hours in a steaming chamber with the provision of alcohol into the chamber; secondarily maturing the secondarily steamed ginseng at 35°C or less for 24 hours in a maturing chamber in such a manner that external water is added to the chamber to maintain the water content of the ginseng at 20% or higher; fixing the form of the secondarily matured ginseng by wrapping the secondarily matured ginseng with linen cloth or tying the secondarily matured ginseng with silk thread; further steaming and maturing the ginseng seven times in the same manner as in the secondary steaming step and the secondary maturing step, respectively, to finish a nonary steaming step and a nonary maturing step; and drying the nonarily matured ginseng in the sun in such a manner that the ginseng has a water content of 14%.

In this method, the primarily steaming step is conducted in the steaming chamber, thesteaming chamber being maintained at a pressure of 0 kg/cm3 ~ 1 kg/cm2 and being changed in temperature from 88 ~ 96°C within 2.5 ~ 4 hours depending on the thickness of the ginseng.

Provided that it is 100% pure, the alcohol is provided in an amount of 5 ~ 10 % into the steaming chamber in all nine rounds of steaming.

Preferably, each maturing step from the primarily maturing step to the nonarily maturing step is conducted immediately after the corresponding steaming step so as to prevent the loss of effective saponin ingredients attributable to carbonization, which may occur during the steaming steps.

In accordance with another aspect of the present invention, there is provided a method for manufacturing a black ginseng extract, comprising: cleaning raw fresh ginseng with water carefully so as not to injure the integrity and ingredients of the fresh ginseng; primarily steaming the cleaned ginseng at 88 ~ 96°C for 2.5 ~ 4 hours with the ginseng's own water in a steaming chamber with alcohol provided into the chamber; primarily maturing the ginseng at 35°C or lower for 24 ~ 72 hours in a maturing chamber with no additional provision of water to the chamber in such a manner that the water content of the ginseng is not decreased to less than 20%; secondarily steaming the primarily matured ginseng at 65 ~ 75°C for 1.5 ~ 2.5 hours in a steaming chamber with the provision of alcohol into the chamber; secondarily maturing the secondarily steamed ginseng at 35°C or less for 24 hours in a maturing chamber in such a manner that external water is added to the chamber to maintain the water content of the ginseng at 20% or higher; fixing the form of the secondarily matured ginseng by wrapping the secondarily matured ginseng with linen cloth or tying the secondarily matured ginseng with silk thread; further steaming and maturing the ginseng seven times in the same manner as in the secondarily steaming step and the secondarily maturing step, respectively, to finish nine rounds of steaming and maturing in total; immersing the ginseng three times in 70% alcohol at 75°C or less to give a rough extract including effective saponin ingredients; filtering the rough extract through a microfilter to remove impurities other than effective saponin ingredients to give a filtrate; concentrating the filtrate in a concentrator which is evacuatedso that the evaporation point of the solvent used in the extracting step is kept at 45°C or less, the concentrator being heated at 75°C or less; dissolving the concentrate in 70% alcohol and removing impurities from the solution through precipitation; and concentrating the solution to the extent that its solid content increases to 60% or higher.

In accordance with a further aspect of thepresent invention, there is provided a method for manufacturing a black ginseng extract, comprising: immersing the black ginseng prepared according to the method three times in 70% alcohol at 75°C or less to give a rough extract including effective saponin ingredients; filtering the rough extract through a microfilter to remove impurities other than effective saponin ingredients to give a filtrate; concentrating the filtrate in a concentrator which is evacuated so that the evaporation point of the solvent used in the extracting step is maintained at 45°C or less, the concentrator being heated to 75°C or less; dissolving the concentrate in 70% alcohol and removing impurities from the solution through precipitation; and concentrating the solution to the extent that the solid content thereof increases to 60% or higher.

Preferably, the black ginseng extract contains effective saponin ingredients including ginsenoside Rg1, ginsenoside Rg2, ginsenoside Rg3, ginsenoside Rd, ginsenoside Re, ginsenoside Rf, ginsenoside Rb1, ginsenoside Rb2, and ginsenoside Rc in an amount of 80 mg/g - 300 mg/g.

The black ginseng is in the form of whole fresh ginseng, in the form of a root body devoid of root hairs, or in the form of root hairs removed from the root body thereof.

In accordance with a still further aspect of the present invention, there is provided a method for manufacturing honey-soaked ginseng confectionary, comprising: cleaning raw fresh ginseng with water to remove impurities from the surface thereof carefully so asnot to injure the integrity and ingredients of the fresh ginseng selecting whole fresh ginseng and dehydrating the surface of the selected ginseng cutting the ginseng into pieces, peeling the ginseng or destroying the surface of the ginseng neatly arranging the ginseng pieces or the whole ginseng on a sieve soaking the ginseng in honey; primarily steaming the honey-coated ginseng at 88 - 96°C for 2.5 ~ 4 hours with the water contained therein; primarily maturing the primarily steamed ginseng in a maturing chamber at a temperature less than 35C for 8 hours; soaking the primarily matured ginseng in honey; secondarily steaming the soaked ginseng at 65 - 75°C for 1.5 - 2.5 hours; secondarily maturing the secondarily steamed ginseng in a maturing chamber at a temperature less than 35°C for 8 hours; further soaking, steaming and maturing the ginseng seven times in the same manner as in the above soaking step, the secondarysteaming step and the secondary maturing step, respectively, to finish nine rounds of steaming and maturing in total; semi-drying the nonarily matured ginseng pruning the semi-dried ginseng drying the pruned ginseng to the extent that the water content of the ginseng meets a predetermined standard; packaging the dried ginseng into vacuum packs; and sterilizing the ginseng in vacuum packs through thermal exposure at 90°C for 30 min.

In this method, no external water is added in any of the eight rounds from the secondary steaming step to the nonary steaming step.

### Advantageous Effects

The black ginseng products, including black ginseng itself, black ginseng extracts and honey-soaked black ginseng confectionary, manufactured according to the methods of the present inventioncan be a commodity competing with conventional ginseng and red ginseng products, and has an excellent taste and flavor for persons of all ages all over the world, in addition to being is healthful for the body.

Also, the natural maturation, instead of drying, subsequent to the steaming processes, allows the ginseng materialto retain a constant level of water therein, thereby preventing the loss of effective saponin ingredients attributable to carbonization,which may occur during the steaming process. According to the present invention, whole black ginseng can be obtained. Further, the provision of alcohol during the steaming processes results in an increase in the value of the final product. Sun drying guarantees the uniform distribution of water throughout the root body and hairs of the black ginseng.

The black ginseng extract manufactured according to the method of the present invention contains effective saponin components in an amount of 80 mg/g - 300 mg/g.

In the honey-soaked black ginseng confectionary, the effective ingredients of the starting material are retained because, after being applied to the black ginseng, honey is completely absorbed into the body of the black ginseng during the steaming process. Also, the repetition of the steaming process in a suitable condition confers the honey-soaked black ginseng confectionary with characteristic and excellent tastes and flavors.

### Best Mode for Carrying Out the Invention

Black ginseng is prepared according to the processes described below.

1. First process: Material Cleaning

Intact fresh ginseng is carefully cleaned with water lest the integrity and ingredients thereof be lost. Foreign mattershould be removed from the surface of fresh ginseng using clean water. However, if fresh ginseng is cleaned too much, it is likely to be damaged, resulting in the loss of effective ingredients and degradation in quality.

After washing, the ginseng roots are sorted according to material statuses, including quality and thickness, because optimal processing conditions, including time periods and temperatures, may change with the quality and status of the material to be treated. Fundamentally, ginseng roots are processed whole: if this is unsuitable, ginseng roots may be divided into bodies and hairs, which are then processed separately.

2. Second Process: Primary Steaming

After the completion of the first process, the material is steamed with its own water content at 88-96°C for 2.5~4 hours in a steam chamber while alcohol is fed into the steam chamber.

According to the present invention, nine rounds of steaming processes are performed for the preparation of black ginseng. In this primary steaming process, additional water is not supplied, but the water retained by the fresh ginseng is used. The water content of fresh ginseng is usually about 70%. If water is externally supplied to the steaming chamber, the resulting excessive water is condensed in the material, and extracts ingredients from the material.

In addition, the steaming time and temperature may be changed within the ranges of 2.5~4 hours and 88-96°C, depending on the thickness of the material. During steaming, the steam chamber is closed, and the pressure therein is maintained at 0 - 1 kg/cm3in order to maximize thermal efficiency and prevent the loss of water at such a high temperature.

In every round of steaming, alcohol is supplied into the steaming chamber in order to prevent degradation attributable to germs throughout the entire procedure and maximize the quality of the material. As a result, the black ginseng prepared according to the method of the present invention exhibits characteristic deep tastes and flavors which cannot be obtained from fresh ginseng, white ginseng or red ginseng.

Preferably, the alcohol is ethanol. When it is 100% pure, ethanol is supplied in an amount of 5-10 % by weight based on the weight of the material.

3. Third Process: Primary Maturing

After completion of the second process, the materialis not dried, but is allowed to undergo maturation in a maturation chamber without the supply of additional water.For natural maturation, the material is left at 35°C or lower for 24-72 hours in order that its water content is not decreased below 20%.

4. Fourth Process: Secondary Steaming

While external water and alcohol are supplied into the steaming chamber, the primarily matured material is steamed at 65-75°C for 1.5~2.5 hours in the steaming chamber.When it is 100% pure, the alcohol is preferably supplied in an amount of 5~10 % by weight based on the weight of the material.

This process is quite different from the second process.Whereas the primary steaming is performed using the water retained by the material, external water is supplied into the chamber from the secondary steaming process.Because the primarily matured material has low water content, if it is heated without the supply of external water, it is not steamed, but is likely to dry out and thus undergo carbonization, incurring ingredient loss and degradation of quality.

The term "carbonization", as used herein, means the decomposition or chemical change of ginseng components into carbons.

5. Fifth Process: Secondary Maturing

Even if it differs from one material to another, the water content is measured to amount to about 40% in the body and about 30% in root hairs. The ginseng material is allowed to mature at 35°C or lower for about 24 hours in a maturation chamber in order that the water content is not decreased to less than 20%. If the roots become too dry at the edges, additional moisture can be provided to the chamber.

6. Sixth Process: Form Fixing

In order to maintain the neat arrangement thereof, the material is wrapped with linen cloth or tied up with silk thread. The linen cloth or silk thread is removed after the final maturing and drying process.

A lot of manual treatment is required to increase the value of the final product. After undergoing the maturation process, the material is much lower in water content than fresh ginseng. Also, the material has a smooth texture so that it can be easily arranged into a neat form.

7. Seventh Process: Tertiary ~ Nonary Steaming and Tertiary - Nonary Maturing

After completion of the sixth process, the material is steamed and matured seven times more in the same manner as in the secondary steaming process and the secondary maturing process, respectively.

8. Eighth Process: Drying

After the nonary maturing process, the material is dried under the sun so that its water content is maintained at 14% or less. This drying process is very important. Even a slight mistake in this drying process results in a great loss of labor, time and money.

After the nine rounds of steaming and maturing processes, the water content of the material (root body and root hairs) is near the criterion of 14%.Therefore, after the nonary maturing process, the drying process is preferably conducted by sun drying.

When sun drying is employed, it takes a long time to dry the material to the desired level of water content, but as long as care is taken to prevent carbonization attributable to excessive drying, the repetition of a process of absorbing air and water into the material and releasing it from the material makes it possible to safely maintain the material at a desired water content level.

Sun drying is a natural drying process that uses sun and air. In contrast, fire drying is an artificial drying process that uses a dryer based on heat and forced air.

After completion of the eighth process, the black ginseng thus produced is unwrapped or untied and selected according to the purpose thereof. If it is measured to have a water content higher than the criterion, the black ginseng is returned to the drying process. Following the selection, the resulting products are inspected and sanitarily packaged according to product form and quality.

Upon packaging, the products thus obtained are classified according to quality state. For example, the products are classified into black ginseng, neat black ginseng and black ginseng rootlet.

As used herein, the term "black ginseng"is intended to refer to whole black ginseng, the term "neat black ginseng" to a black ginseng root from which root hairs have been removed, and the term "black ginseng rootlet" to hairs or rootlets of black ginseng, which have been removed from main roots.

The black ginseng thus produced exhibits unique and deep tastes and flavors characteristic to the black ginseng, which cannot be obtained from fresh ginseng, white ginseng or red ginseng.

From the black ginseng thus obtained, a black ginseng extract can be prepared.

The black ginseng extract according to the present invention is prepared as follows. The material which has undergone the seventh process, that is, the nine rounds of steaming and maturing processes, is utilized.

8. Eighth Process: Extracting

Effective saponin ingredients are extracted from the black ginseng using a 70% alcohol solution. The extraction is repeated three times at 75°C or lower.

9. Ninth Process: Filtering

The extract is filtered through a microfilter to remove matter other than effective saponin ingredients.

10. Tenth Process: Concentrating

The filtrate is concentrated using a concentrator which is evacuated so that the evaporation point of the solvent used in the extracting process is kept at 45°C or less while being heated at 75°C or less.

This temperature condition is specified to prevent the thermal degradation of the effective saponin ingredients and preserve the taste and flavor ingredients retained by the extract.

11. Eleventh Process: Purifying

Using a precipitation method, impurities are removed from a solution of the concentrate in a 70% alcohol solution.

12. Twelfth Process: Concentrating

After the removal of impurities, the solution is concentrated to the extent that its solid content increases to 60% or higher. The resulting concentrate is used as an extract.

The concentration of the solution is performed in a concentrator which is evacuated so that the evaporation point of the solvent used in the extracting process is kept at 45°C or less while being heated at 75°C or less. This temperature condition is set forth to prevent the thermal degradation of the effective saponin ingredients and preserve the taste and flavor ingredients retained by the extract.

The black ginseng extract thus produced retains the taste and flavor characteristic of black ginseng, and contains effective saponin in an amount of 80 mg/g - 300 mg/g.

The black ginseng extract is inspected according to a standard testing method in an authorized facility, and is sanitarily packaged into predetermined units.

As described above, the black ginseng material which has undergone the procedure from the first to the seventh process can be used to prepare the black ginseng extract according to the present invention, and this extraction procedure utilizing the black ginseng material after the seventh process has fewer steps than the *de novo* procedure which starts with fresh ginseng, increasing process efficiency.

Alternatively, the black ginseng material obtained after the eighth process of drying may be used to prepare a black ginseng extract. That is, the material which has undergone the nonary steaming process and the nonary maturing process and then the eighth process of drying is subjected to extraction, filtration, concentration, purification and concentration, in that order, as in the foregoing.

The extraction, filtration, concentration, purification and concentration processes are performed in the same manner as in the above method of preparing black ginseng extract.

In detail, the alternative extraction method for the preparation of black ginseng extract is performed as follows.

Material (raw fresh ginseng)→ 1. First process: washing → 2. Second process: primary steaming → 3. Third process: primary maturing → 4. Fourth process: secondary steaming → 5. Fifth process: secondary steaming → 6. Sixth process: form fixing → 7. Seventh process: tertiary - nonary steaming and tertiary → nonary maturing → 8. Eighth process: drying → 9. Ninth process: extracting → 10. Tenth process: filtering → 11. Eleventh process: concentrating → 12. Twelfth process: purifying → 13. Thirteenth process: concentrating

After the thirteenth process, the black ginseng extract is inspected according to a standard testing method in an authorized facility, and is sanitarily packaged into predetermined units.

The black ginseng extract prepared according to the method of the present invention was found to contain all of the effective saponin ingredients of red ginseng given in Table 1, below, as the result of analysis by an authorized organization.

**Table 1**

| | | |
|---|---|---|
| Ginsenoside-Rf | Ginsenoside-Re | Ginsenoside-Rd |
| Ginsenoside-Rc | Ginsenoside-Rb2 | Ginsenoside-Rb1 |

In the black ginseng extract prepared according to the present invention, the effective saponin ingredients including ginsenoside Rg1, ginsenoside Rg2, ginsenoside Rg3, ginsenoside Rd, ginsenoside Re, ginsenoside Rf, ginsenoside Rb1, ginsenoside Rb2, and ginsenoside Rc are present in an amount of 80 mg/g - 300 mg/g.

Also, the black ginseng may be used to prepare honey-soaked black ginseng confectionary as follows.

1. First process: Cleaning

Fresh ginseng is used as a material. Because it is the major determinant of the quality of the final product, the material must be strictly selected from among good kinds of ginseng, exclusive of the following defective kinds of ginseng.

Yellow ginseng refers to ginseng whose surface is not neat due to a problematic growth procedure, and shows a significantly rough reddish yellow color. The surface of the ginseng appears normal in surface color, but is found to be roughly pockmarked and have injured inner surfaces when scrutinized.

Rotten ginseng looks normal, but is flabby and has black scars at the edges of the core. It smells rotten. Tousled ginseng refers to deformed ginseng which has too short a root body and too many root hairs.

Dormant ginseng has no normal rhizomes and stems because its leaves have fallen early, or because new annual leaves have not yet sprouted. In such ginseng, there are no substantial amounts of effective ingredients and cell tissues. Thus, dormant ginseng does not provide effective ingredients when used in the preparation of black ginseng extracts.

The material is cleaned to remove impurities from the surface thereof, with caution not to harm the integrity thereof.

After being selected from among the cleaned fresh ginseng, the material in whole form is dried to the extent that moisture is eliminated from the surface thereof.

Selection with the naked eye is easier after the ginseng is cleaned than before. The cleaned ginseng is inspected for defects and sorted into those which are to be processed in their integrity and divided parts.

2. Second Process: Surface Dehydration

There are many reasons for dehydrating the surface of the selected materials. For example, the materials cannot be preserved for a long period of time unless their surface is dry: otherwise, they are prone to spoil. The water on material surfaces acts as an impediment to subsequent processes. Also, when the material is soaked in honey, the water on the surface thins the honey, decreasing the efficacy of the process.

3. Third Process: Material Cutting and Peeling

In this process, the material is peeled and/or cut into pieces having a desired size. Alternatively, the bark may be destroyed.

The material, having a dry surface, may be cut into pieces which are formed into honey-soaked ginseng confectionary, or may be used together with the preparation of honey-soaked ginseng confectionary. In the latter case, because the skin of the ginseng plays a role of inhibiting the takeup of honey or sugar by the ginseng, it must be peeled off from the material or destroyed so as to make it easy to introduce honey or sugar into the body of the ginseng.

4. Fourth Process: Neatly Arranging on Sieve

Pieces of the material are arranged neatly on a sieve.

This process of neatly arranging the material pieces is indispensable in order to increase the efficacy of the method and the value of the final products.

5. Fifth Process: Honey Soaking

The materials are soaked in honey. Conventionally, the ginseng materials are soaked in honey, followed by boiling the honey to absorb honey into the body of the ginseng. However, boiling causes the effective ingredients of ginseng to be released externally, so that the final product is degraded. In the present invention, the ginseng material is merely soaked in honey, without heating.

6. Sixth Process: Primary Steaming

The material, uniformly coated with honey, is steamed at 88 - 96°C for 2.5 - 4 hours using the water retained by the material.

7. Seventh Process: Primary Maturing

In a maturation chamber, the primarily steamed material is primarily matured at 35°C or lower for 8 hours.

8. Eighth Process: Honey Soaking

The primarily matured material is soaked in honey.

9. Ninth Process: Secondary Steaming

The material having undergone the eighth process is steamed at 65 - 75°C for 1.5 - 2.5 hours with no provision of additional water.

10. Tenth Process: Secondary Maturing

The secondarily steamed material is matured at a temperature lower than 35°C for 8 hours.

11. Eleventh Process: Honey Soaking, Tertiary ~ Nonary Steaming and Tertiary ~ Nonary Maturing

After being soaked in honey again, the material is subjected to seven additional steaming and maturing processes in the same manner as in the secondary steaming process and the secondary maturing process, respectively.

The steps from the secondary steaming process to the nonary steaming process are performed with no provision of additional water.

12. Twelfth Process: Primary Drying

After the eleventh process, the material is partially dried.

The resulting semi-dried material is easy to prune.

13. Thirteenth Process: Material Pruning

The semi-dried material obtained after the twelfth process is pruned. This process aims to increase the commercial value of the products.

14. Fourteenth Process: Secondary Drying

Following the thirteenth process, the material is dried to the extent that the water content meets the standard.

15. Fifteenth Process: Packaging

The secondarily dried materials are packaged into vacuum packs.

After the secondary drying process, the materials are inspected for whether they meet the standards or not, and the materials chosen as suitable are packaged into vacuum packs.

16. Sixteenth Process: Sterilizing

The materials packaged in vacuum packs are thermally exposed at 90°C for 30 min. The black ginseng thus produced is found to have a delicate taste and flavor with a smooth and chewy texture.

## Claims

1. A method for preparing black ginseng from fresh ginseng, comprising:
cleaning raw fresh ginseng with water carefully, so as not to injure the integrity and ingredients of the fresh ginseng;
primarily steaming the cleaned ginseng at 88 - 96°C for 2.5 ~ 4 hours with the ginseng's own water in a steaming chamber with alcohol provided to the chamber;
primarily maturing the ginseng at 35°C or lower for 24 - 72 hours in a maturing chamber with no additional provision of water to the chamber in a manner such that the water content of the ginseng is not decreased below 20%;
secondarily steaming the primarily matured ginseng at 65 - 75°C for 1.5 ~ 2.5 hours in a steaming chamber with the provision of alcohol into the chamber;
secondarily maturing the secondarily steamed ginseng at 35°C or less for 24 hours in a maturing chamber in such a manner that external water is added to the chamber to maintain the water content of the ginseng at 20% or higher;
fixing a form of the secondarily matured ginseng by wrapping the secondarily matured ginseng with linen cloth or tying the secondarily matured ginseng with silk thread;
further steaming and maturing the ginseng seven times in the same manners as in the secondary steaming step and the secondary maturing step, respectively, to finish a nonarily steaming step and a nonarily maturing step; and
drying the nonarily matured ginseng in the sun in such a manner that the ginseng has a water content of 14%.

2. The method as set forth in claim 1, wherein the primary steaming step is conducted in the steaming chamber, said steaming chamber being maintained at a pressure of 0 kg/cm³ ~ 1kg/cm², and being changed in temperature from 88 - 96°C within 2.5 - 4 hours depending on the thickness of the ginseng.

3. The method as set forth in claim 1 or 2, wherein, provided that it is 100% pure, the alcohol is provided in an amount of 5 - 10 % into the steaming chamber in all of the nine rounds of the steaming.

4. The method as set forth in anyone or more of claims 1 to 3, wherein each of the primary maturing step to the nonary maturing step is conducted immediately after the corresponding steaming step so as to prevent the loss of effective saponin ingredients attributable to carbonization, which may occur during the steaming steps.

5. A method for preparing a black ginseng extract, comprising:
cleaning raw fresh ginseng with water carefully so as not to injure the integrity and ingredients of the fresh ginseng;
primarily steaming the cleaned ginseng at 88 - 96°C for 2.5 ~ 4 hours with the ginseng's own water in a steaming chamber, with alcohol provided into the chamber;
primarily maturing the ginseng at 35°C or lower for 24 - 72 hours in a maturing chamber with no additional provision of water to the chamber in a manner such that the water content of the ginseng is not decreased to less than 20%;
secondarily steaming the primarily matured ginseng at 65 - 75°C for 1.5 - 2.5 hours in a steaming chamber with the provision of alcohol into the chamber;
secondarily maturing the secondarily steamed ginseng at 35°C or less for 24 hours in a maturing chamber in such a manner that external water is added to the chamber to maintain the water content of the ginseng at 20% or higher;
fixing a form of the secondarily matured ginseng by wrapping the secondarily matured ginseng with linen cloth or tying the secondarily matured ginseng with silk thread;
further steaming and maturing the ginseng seven times in the same manner as in the secondary steaming step and the secondary maturing step, respectively, to finish nine rounds of steaming and maturing in total;
immersing the ginseng three times in 70% alcohol at 75°C or less to give a rough extract including effective saponin ingredients;
filtering the rough extract through a microfilter to remove impurities other than effective saponin ingredients, to give a filtrate;
concentrating the filtrate in a concentrator which is evacuated so that an evaporation point of a solvent used in the extracting step is kept at 45°C or less,
the concentrator being heated to 75°C or less;
dissolving the concentrate in 70% alcohol and removing impurities from the solution through precipitation; and
concentrating the solution to an extent that its solid content increases to 60% or higher.

6. A method for preparing a black ginseng extract, comprising:
immersing the black ginseng prepared according to the method of claim 1 three times in 70% alcohol at 75°C or less to give a rough extract including effective saponin ingredients;
filtering the rough extract through a microfilter to remove impurities other than effective saponin ingredients to give a filtrate;
concentrating the filtrate in a concentrator which is evacuated so that the evaporation point of a solvent used in the extracting step is maintained at 45°C or less, the concentrator being heated to 75°C or less;
dissolving the concentrate in 70% alcohol and removing impurities from the solution through precipitation; and
concentrating the solution to the extent that its solid content increases to 60% or higher.

7. The method as set forth in claim 5 or 6, wherein the black ginseng extract contains effective saponin ingredients including ginsenoside Rg1, ginsenoside Rg2, ginsenoside Rg3, ginsenoside Rd, ginsenoside Re, ginsenoside Rf, ginsenoside Rb1, ginsenoside Rb2, and ginsenoside Rc in an amount of 80 mg/g ~ 300 mg/g.

8. The method as set forth in anyone or more of claims 1 to 7, wherein the black ginseng is in a whole form of fresh ginseng, in a form of a root body devoid of root hairs, or in a form of root hairs removed from the root body thereof.

9. A method for preparing honey-soaked ginseng confectionary, comprising:
cleaning raw fresh ginseng with water to remove impurities from the surface thereof carefully, so as not to injure the integrity and ingredients of the fresh ginseng;
selecting fresh ginseng in whole form and dehydrating the surface of the selected ginseng;
cutting the ginseng into pieces, peeling the ginseng, or destroying the surface of the ginseng;
neatly arranging the ginseng pieces or the whole ginseng in their integrity on a sieve;
soaking the ginseng in honey;
primarily steaming the honey-coated ginseng at 88 ~ 96°C for 2.5 ~ 4 hours with the water contained therein;
primarily maturing the primarily steamed ginseng in a maturing chamber at a temperature less than 35°C for 8 hours;
soaking the primarily matured ginseng in honey;
secondarily steaming the soaked ginseng at 65 ~ 75°C for 1.5 ~ 2.5 hours;
secondarily maturing the secondarily steamed ginseng in a maturing chamber at a temperature less than 35°C for 8 hours;
further soaking, steaming and maturing the ginseng seven times in the same manner as in the above soaking step, the secondary steaming step and the secondary maturing step, respectively, to finish nine rounds of steaming and
maturing in total;
semi-drying the nonarily matured ginseng;
pruning the semi-dried ginseng;
drying the pruned ginseng to the extent that the water content of the ginseng meets a predetermined standard;
packaging the dried ginseng into vacuum packs; and
sterilizing the ginseng in vacuum packs by thermal exposure at 90°C for 30 min.

10. The method as set forth in claim 9, wherein no external water is added in any of the eight rounds of the secondary steaming step to the nonary steaming step.

## Patentansprüche

1. Verfahren zum Zubereiten von schwarzem Ginseng aus frischem Ginseng, aufweisend:
- vorsichtiges Reinigen von rohem, frischem Ginseng mit Wasser, um die Unversehrtheit und Inhaltsstoffe des frischen Ginseng nicht zu beschädigen;
- primäres Dämpfen des gereinigten Ginseng bei 88°C bis 96°C für 2,5 bis 4 Stunden mit dem ginsengeigenen Wasser in einer Dämpfungskammer mit Alkohol, der der Kammer bereitgestellt wird;
- primäres Reifen des Ginseng bei 35°C oder weniger für 24 bis 72 Stunden in einer Reifungskammer ohne zusätzliche Bereitstellung von Wasser zur Kammer derart, dass der Wassergehalt des Ginseng nicht unter 20% reduziert wird;
- sekundäres Dämpfen des primär gereiften Ginseng bei 65°C bis 75°C für 1,5 bis 2,5 Stunden in einer Dämpfungskammer mit der Bereitstellung von Alkohol in die Kammer;
- sekundäres Reifen des sekundär gedämpften Ginseng bei 35°C oder weniger für 24 Stunden in einer Reifungskammer derart, dass externes Wasser zur Kammer hinzugefügt wird, um den Wassergehalt des Ginseng bei 20% oder höher zu erhalten;
- Fixieren einer Form des sekundär gereiften Ginseng durch Umwickeln des sekundär gereiften Ginseng mit Leinentuch oder Zusammenbinden des sekundär gereiften Ginseng mit Seidengarn;
- weiteres siebenmaliges Dämpfen und Reifen des Ginseng in den gleichen Weisen wie in der sekundären Dämpfungsstufe beziehungsweise in der sekundären Reifungsstufe, um eine nonäre Dämpfungsstufe und eine nonäre Reifungsstufe zu beenden; und
- Trocknen des nonär gereiften Ginseng in der Sonne derart, dass der Ginseng einen Wassergehalt von 14% hat.

2. Verfahren nach Anspruch 1, wobei die primäre Dämpfungsstufe in der Dämpfungskammer durchgeführt wird, wobei die Dämpfungskammer bei einem Druck von 0 kg/cm³ bis 1 kg/cm² gehalten und innerhalb von 2,5 bis 4 Stunden, abhängig von der Dicke des Ginseng, hinsichtlich einer Temperatur von 88°C auf 96°C geändert wird.

3. Verfahren nach Anspruch 1 oder 2, wobei der Alkohol, vorausgesetzt, dass er 100% rein ist, in allen neun Durchgängen des Dämpfens in einem Ausmaß von 5% bis 10% in die Dämpfungskammer bereitgestellt wird.

4. Verfahren nach einem oder mehreren der Ansprüche 1 bis 3, wobei jede der primären bis nonären Reifungsstufe unmittelbar nach der entsprechenden Dämpfungsstufe durchgeführt wird, um einen Verlust von wirksamen Saponin-Inhaltsstoffen zu verhindern, der einer Verkohlung zurechenbar ist, die während der Dämpfungsstufen auftreten kann.

5. Verfahren zum Zubereiten von schwarzem Ginsengextrakt, aufweisend:
- Vorsichtiges Reinigen von rohem, frischem Ginseng mit Wasser, um eine Unversehrtheit und Inhaltsstoffe des frischen Ginseng nicht zu beschädigen;
- primäres Dämpfen des gereinigten Ginseng bei 88°C bis 96°C für 2,5 bis 4 Stunden mit dem ginsengeigenen Wasser in einer Dämpfungskammer mit Alkohol, der in die Kammer bereitgestellt wird;
- primäres Reifen des Ginseng bei 35°C oder weniger für 24 bis 72 Stunden in einer Reifungskammer ohne zusätzliche Bereitstellung von Wasser in die Kammer derart, dass der Wassergehalt des Ginseng nicht unter 20% reduziert wird;
- sekundäres Dämpfen des primär gereiften Ginseng bei 65°C bis 75°C für 1,5 bis 2,5 Stunden in einer Dämpfungskammer mit der Bereitstellung von Alkohol in die Kammer;
- sekundäres Reifen des sekundär gedämpften Ginseng bei 35°C oder weniger für 24 Stunden in einer Reifungskammer derart, dass externes Wasser zur Kammer hinzugefügt wird, um den Wassergehalt des Ginseng bei 20% oder höher zu erhalten;
- Fixieren einer Form des sekundär gereiften Ginseng durch Umwickeln des sekundär gereiften Ginseng mit Leinentuch oder Zusammenbinden des sekundär gereiften Ginseng mit Seidengarn;
- weiteres siebenmaliges Dämpfen und Reifen des Ginseng in der gleichen Weise wie in der sekundären Dämpfungsstufe beziehungsweise in der sekundären Reifungsstufe, um insgesamt neun Durchgänge von Dämpfen und Reifen zu beenden;
- dreimaliges Eintauchen des Ginseng in 70% Alkohol bei 75°C oder weniger um ein grobes Extrakt mit wirksamen Saponin-Inhaltsstoffen zu ergeben;
- Filtern des groben Extrakts durch ein Mikrofilter, um Verunreinigungen mit Ausnahme von wirksamen Saponin-Inhaltsstoffen zu entfernen, um ein Filtrat zu ergeben;
- Verdichten des Filtrats in einem luftleeren Verdichter derart, dass ein Verdampfungspunkt eines Lösungsmittels, das in der Extraktionsstufe verwendet wird, bei 45°C oder weniger gehalten wird, wobei der Verdichter auf 75°C oder weniger erwärmt wird;
- Auflösen des Konzentrats in 70% Alkohol und Entfernen von Verunreinigungen aus der Lösung durch Ausfällung; und
- Verdichten der Lösung in einem Ausmaß, dass ihr Feststoffgehalt auf 60% oder höher ansteigt.

6. Verfahren zum Zubereiten von schwarzem Ginsengextrakt, aufweisend:
- dreimaliges Eintauchen des nach dem Verfahren gemäß Anspruch 1 zubereiteten schwarzen Ginseng in 70% Alkohol bei 75°C oder weniger, um ein grobes Extrakt mit wirksamen Saponin-Inhaltsstoffen zu ergeben;
- Filtern des groben Extrakts durch ein Mikrofilter, um Verunreinigungen mit Ausnahme von wirksamen Saponin-Inhaltsstoffen zu entfernen, um ein Filtrat zu ergeben;
- Verdichten des Filtrats in einem luftleeren Verdichter derart, dass der Verdampfungspunkt eines Lösungsmittels, das in der Extraktionsstufe verwendet wird, bei 45°C oder weniger gehalten wird, wobei der Verdichter auf 75°C oder weniger erwärmt wird;
- Auflösen des Konzentrats in 70% Alkohol und Entfernen von Verunreinigungen aus der Lösung durch Ausfällung; und
- Verdichten der Lösung soweit, dass ihr Feststoffgehalt auf 60% oder höher ansteigt.

7. Verfahren nach Anspruch 5 oder 6, wobei der schwarze Ginsengextrakt wirksame Saponin-Inhaltsstoffe enthält, einschließlich Ginsenosid Rg1, Ginsenosid Rg2, Ginsenosid Rg3, Ginsenosid Rd, Ginsenosid Re, Ginsenosid Rf, Ginsenosid Rb1, Ginsenosid Rb2, und Ginsenosid Rc in einem Ausmaß von 80mg/g bis 300mg/g.

8. Verfahren nach einem oder mehreren der Ansprüche 1 bis 7, wobei der Ginseng in einer ganzen Form von frischem Ginseng, in einer Form eines Wurzelkörpers ohne Wurzelhaare, oder in einer Form von Wurzelhaaren, die vom Wurzelkörper entfernt sind, ist.

9. Verfahren zum Zubereiten von honiggetränktem Ginsengkonfekt, aufweisend:
- Reinigen von rohem, frischem Ginseng mit Wasser, um Verunreinigungen von der Oberfläche davon sorgfältig zu entfernen, um eine Unversehrtheit und Inhaltsstoffe des frischen Ginseng nicht zu beschädigen;
- Auswählen von frischem Ginseng im Ganzen und Dehydrieren der Oberfläche des ausgewählten Ginseng;
- Schneiden des Ginseng in Stücke, Enthäuten des Ginseng, oder Zerstören der Oberfläche des Ginseng;
- übersichtliches Anordnen der Ginsengstücke oder des ganzen Ginseng in ihrer Vollständigkeit auf einem Sieb;
- Tränken des Ginseng in Honig;
- primäres Dämpfen des honigbeschichteten Ginseng bei 88°C bis 96°C für 2,5 bis 4 Stunden mit dem darin enthaltenen Wasser;
- primäres Reifen des primär gedämpften Ginseng in einer Reifungskammer bei einer Temperatur von weniger als 35°C für 8 Stunden;
- Tränken des primär gereiften Ginseng in Honig;
- sekundäres Dämpfen des getränkten Ginseng bei 65°C bis 75°C für 1,5 bis 2,5 Stunden;
- sekundäres Reifen des sekundär gedämpften Ginseng in einer Reifungskammer bei einer Temperatur von weniger als 35°C für 8 Stunden;
- weiteres siebenmaliges Tränken, Dämpfen und Reifen des Ginseng in derselben Weise wie in der obigen Tränkungsstufe, beziehungsweise in der sekundären Dämpfungsstufe, beziehungsweise in der sekundären Reifungsstufe, um insgesamt neun Durchgänge von Dämpfen und Reifen zu beenden;
- Halbtrocknen des nonär gereiften Ginseng;
- Schneiden des halbgetrockneten Ginseng;
- Trocknen des geschnittenen Ginseng soweit, dass der Wassergehalt des Ginseng einen vorbestimmten Standard erfüllt;
- Verpacken des getrockneten Ginseng in Vakuumverpackungen; und
- Sterilisieren des Ginseng in Vakuumverpackungen durch thermische Einwirkung bei 90°C für 30 Minuten.

10. Verfahren nach Anspruch 9, wobei in jedem der acht Durchgänge der sekundären Dämpfungsstufe bis zur nonären Dämpfungsstufe kein externes Wasser hinzugefügt wird.

## Revendications

1. Procédé pour préparer du ginseng noir à partir de ginseng frais, comprenant :
le nettoyage soigneux de ginseng frais brut avec de l'eau, de façon à ne pas nuire à l'intégrité et aux ingrédients du ginseng frais ;
un premier étuvage du ginseng nettoyé, à une température de 88 à 96°C pendant 2,5 à 4 heures, avec la propre eau du ginseng dans une chambre d'étuvage, de l'alcool étant introduit dans la chambre ;
une première maturation du ginseng à 35°C ou moins pendant 24 à 72 heures dans une chambre de maturation sans apport supplémentaire d'eau dans la chambre de telle manière que la teneur en eau du ginseng ne diminue pas en-deçà de 20 % ;
un deuxième étuvage du ginseng ayant subi la première maturation, à une température de 65 à 75°C pendant 1,5 à 2,5 heures dans une chambre d'étuvage avec introduction d'alcool dans la chambre ;
une deuxième maturation du ginseng ayant subi le deuxième étuvage, à 35°C ou moins pendant 24 heures dans une chambre de maturation de telle manière que de l'eau soit ajoutée dans la chambre depuis l'extérieur pour maintenir la teneur en eau du ginseng à 20 % ou plus ;
la fixation d'une forme du ginseng ayant subi la deuxième maturation par enveloppement du ginseng ayant subi la deuxième maturation dans une toile de lin ou ficelage du ginseng ayant subi la deuxième maturation avec un fil de soie ;
un nouvel étuvage et une nouvelle maturation du ginseng, sept fois, de la même manière que lors de l'étape de deuxième étuvage et de l'étape de deuxième maturation, respectivement, pour finir par une neuvième étape d'étuvage et une neuvième étape de maturation ; et
le séchage au soleil du ginseng ayant subi la neuvième maturation de telle manière que le ginseng ait une teneur en eau de 14 %.

2. Procédé selon la revendication 1, dans lequel l'étape de premier étuvage est effectuée dans la chambre d'étuvage, ladite chambre d'étuvage étant maintenue sous une pression de 0 kg/cm² à 1 kg/cm², et sa température étant changée de 88-96°C en 2,5 à 4 heures en fonction de l'épaisseur du ginseng.

3. Procédé selon la revendication 1 ou 2, dans lequel, sous réserve que sa pureté soit de 100 %, l'alcool est introduit dans la chambre d'étuvage en une quantité de 5 à 10 % dans tous les neuf tours d'étuvage.

4. Procédé selon l'une quelconque des revendications 1 à 3, dans lequel chacune des étapes de la première maturation à la neuvième maturation est effectuée immédiatement après l'étape d'étuvage correspondante de façon que soit empêchée une perte d'ingrédients actifs de saponine pouvant être attribuée à une carbonisation, qui peut se produire durant les étapes d'étuvage.

5. Procédé pour préparer un extrait de ginseng noir, comprenant :
le nettoyage soigneux de ginseng frais brut avec de l'eau, de façon à ne pas nuire à l'intégrité et aux ingrédients du ginseng frais ;
un premier étuvage du ginseng nettoyé, à une température de 88 à 96°C pendant 2,5 à 4 heures avec la propre eau du ginseng dans une chambre d'étuvage, de l'alcool étant introduit dans la chambre ;
une première maturation du ginseng à 35°C ou moins pendant 24 à 72 heures dans une chambre de maturation sans apport supplémentaire d'eau dans la chambre de telle manière que la teneur en eau du ginseng ne diminue pas en-deçà de 20 % ;
un deuxième étuvage du ginseng ayant subi la première maturation, à une température de 65 à 75°C pendant 1,5 à 2,5 heures dans une chambre d'étuvage avec introduction d'alcool dans la chambre ;
une deuxième maturation du ginseng ayant subi le deuxième étuvage, à 35°C ou moins pendant 24 heures dans une chambre de maturation de telle manière que de l'eau soit ajoutée à la chambre depuis l'extérieur pour maintenir la teneur en eau du ginseng à 20 % ou plus ;
la fixation d'une forme de ginseng ayant subi la deuxième maturation par enveloppement du ginseng ayant subi la deuxième maturation dans une toile de lin ou ficelage du ginseng ayant subi la deuxième maturation avec un fil de soie ;
un nouvel étuvage et une nouvelle maturation du ginseng, sept fois, de la même manière que lors de l'étape de deuxième étuvage et de l'étape de deuxième maturation, respectivement, pour réaliser au total neuf tours d'étuvage et de maturation ;
l'immersion du ginseng, trois fois, dans de l'alcool à 70 % à 75°C ou moins pour obtenir un extrait brut contenant des ingrédients actifs de saponine ;
la filtration de l'extrait brut sur un microfiltre pour éliminer des impuretés autres que les ingrédients actifs de saponine, afin d'obtenir un filtrat ;
la concentration du filtrat dans un concentrateur qui est amené à un vide tel que le point d'évaporation du solvant utilisé dans l'étape d'extraction soit maintenu à 45°C ou moins, le concentrateur étant chauffé à 75°C ou moins ;
la dissolution du concentré dans de l'alcool à 70 % et l'élimination des impuretés dans la solution par précipitation ; et
la concentration de la solution jusqu'à ce que sa teneur en extrait sec augmente à 60 % ou plus.

6. Procédé pour préparer un extrait de ginseng noir, comprenant :
l'immersion du ginseng noir préparé conformément au procédé de la revendication 1, trois fois, dans de l'alcool à 70 % à 75°C ou moins pour obtenir un extrait brut contenant des ingrédients actifs de saponine ;
la filtration de l'extrait brut sur un microfiltre pour éliminer des impuretés autres que les ingrédients actifs de saponine, afin d'obtenir un filtrat ;
la concentration du filtrat dans un concentrateur qui est amené à un vide tel que le point d'évaporation du solvant utilisé dans l'étape d'extraction soit maintenu à 45°C ou moins, le concentrateur étant chauffé à 75°C ou moins ;
la dissolution du concentré dans de l'alcool à 70 % et l'élimination des impuretés dans la solution par précipitation ; et
la concentration de la solution jusqu'à ce que sa teneur en extrait sec augmente à 60 % ou plus.

7. Procédé selon la revendication 5 ou 6, dans lequel l'extrait de ginseng noir contient des ingrédients actifs de saponine comprenant le ginsénoside Rg1, le ginsénoside Rg2, le ginsénoside Rg3, du ginsénoside Rd, le ginsénoside Re, le ginsénoside Rf, le ginsénoside Rb1, le ginsénoside Rb2, et le ginsénoside Rc en une quantité de 80 mg/g à 300 mg/g.

8. Procédé selon l'une quelconque des revendications 1 à 7, dans lequel le ginseng noir est sous la forme de ginseng frais entier, sous la forme de corps de racine exempt de chevelu racinaire, ou sous la forme de chevelu racinaire retiré du corps de racine de celui-ci.

9. Procédé pour préparer une confiserie au ginseng trempé dans du miel, comprenant :
le nettoyage soigneux de ginseng frais brut avec de l'eau pour éliminer les impuretés à la surface de celui-ci, de façon à ne pas nuire à l'intégrité et aux ingrédients du ginseng frais ;
la sélection de ginseng frais sous forme entière et la déshydratation de la surface du ginseng sélectionné ;
le découpage en fragments du ginseng, le pelage du ginseng, ou la destruction de la surface du ginseng ;
la disposition nette des fragments de ginseng ou du ginseng entier, en prenant soin de leur intégrité, sur un tamis ;
l'immersion du ginseng dans du miel ;
un premier étuvage du ginseng enrobé de miel à une température de 88 à 96°C pendant 2,5 à 4 heures avec l'eau qu'il contient ;
une première maturation du ginseng ayant subi le premier étuvage, dans une chambre de maturation à une température inférieure à 35°C pendant 8 heures ;
l'immersion dans du miel du ginseng ayant subi la première maturation ;
un deuxième étuvage du ginseng ayant subi l'immersion, à une température de 65 à 75°C pendant 1,5 à 2,5 heures ;
une deuxième maturation du ginseng ayant subi le deuxième étuvage, dans une chambre de maturation à une température inférieure à 35°C pendant 8 heures ;
une nouvelle immersion, un nouvel étuvage et une nouvelle maturation du ginseng, sept fois, de la même manière que lors de l'étape d'immersion ci-dessus, de l'étape de deuxième étuvage et de l'étape de deuxième maturation, respectivement, pour réaliser au total neuf tours d'étuvage et de maturation ;
le semi séchage du ginseng ayant subi la neuvième maturation ;
l'émondage du ginseng à semi-séché ;
le séchage du ginseng émondé de telle manière que la teneur en eau du ginseng satisfasse à une norme prédéterminée ;
le conditionnement du ginseng séché dans des sachets sous vide ; et
la stérilisation du ginseng dans les sachets sous vide par exposition thermique à 90°C pendant 30 minutes.

10. Procédé selon la revendication 9, dans lequel aucune eau n'est ajoutée depuis l'extérieur dans aucun des huit tours allant de l'étape de deuxième étuvage à l'étape de ne.0uvième d'étuvage.
